# EUROPÄISCHE PATENTSCHRIFT

(11) **EP 2 408 485 B1**
(45) Veröffentlichungstag und Bekanntmachung des Hinweises auf die Patenterteilung: **21.06.2017**
(21) Anmeldenummer: 10711368.0
(22) Anmeldetag: 19.03.2010
(51) Int. Cl.: A61L 2/18, A61L 2/26, A61L 2/07

(54) **GERÄT ZUM DESINFIZIEREN VON ZAHNÄRZTLICHEN INSTRUMENTEN**
DEVICE FOR STERILISING OF DENTAL INSTRUMENTS
DISPOSITIF DE STÉRILISATION D'INSTRUMENTS DENTAIRES

(30) Priorität: 20.03.2009 DE 102009014065; 17.02.2010 DE 102010002030
(43) Veröffentlichungstag der Anmeldung: 25.01.2012
(73) Patentinhaber: Kaltenbach & Voigt GmbH, 88400 Biberach (DE)
(72) Erfinder: HECKENBERGER, Hans, 88433 Aßmannshardt (DE); WIEK, Hans-Dieter, 88454 Hochdorf (DE); LOTT, Herbert, 88410 Bad Wurzach-Arnach (DE); SAUR, Frank, 89073 Ulm (DE); STEMPFLE, Johann, 89284 Pfaffenhofen (DE)
(74) Vertreter: Thun, Clemens
(86) Internationale Anmeldenummer: PCT/EP2010/053604
(87) Internationale Veröffentlichungsnummer: WO 2010/106161

(56) Entgegenhaltungen:
- EP-A1- 1 484 069
- EP-A1- 1 749 502
- EP-A1- 1 749 502
- EP-A1- 1 749 502
- EP-A1- 1 769 721
- EP-A1- 1 769 721
- EP-A2- 1 121 942
- WO-A1-00/57929
- WO-A1-00/57929
- WO-A1-00/59553
- WO-A1-03/084578
- WO-A1-03/084578
- WO-A2-2005/011749
- WO-A2-2005/011749
- DE-A1- 4 127 776
- DE-U1- 9 404 372
- US-A- 5 823 340
- US-A1- 2005 112 040
- Prof. Dr.-Ing. Klaus Strohmeier: "Komponenten des Anlagenbaus" 1. Dezember 2002 (2002-12-01), , München , XP002591370 Seite 154a
- Prof. Dr.-Ing. Klaus Strohmeier: "Beanspruchungsgerechte Anlagensimulation" 1. Dezember 2002 (2002-12-01), , XP002591371 Seite 42h

## Beschreibung

Die vorliegende Erfindung betrifft ein Gerät, welches zum Desinfizieren, Sterilisieren und/oder Pflegen von ärztlichen Instrumenten vorgesehen ist. Insbesondere sollen mit dem Gerät zahnärztliche Instrumente aufbereitet werden.

Bei ärztlichen oder zahnärztlichen Handstücken handelt es sich um rohrförmige Teile, die der Arzt während der Behandlung als Griffhülse ergreift. Ein üblicherweise in der zahnmedizinischen Praxis verwendetes Handstück ist ein sogenanntes Bohrhandstück, das an seinem vorderen Ende ein Behandlungswerkzeug, insbesondere einen Bohrer trägt und mit seinem hinteren Ende mittels einer Kupplung mit einem Versorgungsschlauch gekoppelt ist. Durch das Handstück erstrecken sich Versorgungsleitungen für Energie zum Antrieb des Behandlungsinstruments sowie Fluidleitungen für Behandlungsmedien, beispielsweise Luft und/oder Wasser. Unterschieden wird oftmals zwischen sogenannten Turbinen-Handstücken, bei denen zur Versorgung einer im vorderen Endbereich angeordneten Turbine Druckluft vorgesehen ist, und sogenannten Motor-Handstücken, welche als Antriebseinheit einen Elektromotor aufweisen.

Zur Aufrechterhaltung der Funktion der Handstücke bedarf es von Zeit zu Zeit einer Pflege, insbesondere der drehbar gelagerten Antriebselemente. Ferner führen die in der zahnärztlichen Praxis immer weiter ansteigenden Hygieneanforderungen dazu, dass eine Aufbereitung von Handstücken in regelmäßigen zeitlichen Abständen zu erfolgen hat. Die erfolgreiche Aufbereitung und Einhaltung der entsprechenden Vorgaben muss hierbei durch den Zahnarzt lückenlos dokumentiert werden, was einen nicht unerheblichen personellen und organisatorischen Aufwand nach sich zieht.

Eine manuelle Wiederaufbereitung zahnärztlicher Handstücke erfolgte bislang dadurch, dass die Instrumente nach Gebrauch am Patienten zunächst sprühdesinfiziert und äußerlich abgewaschen wurden. Eine Innenreinigung der Instrumente wurde hingegen in der Regel nicht durchgeführt. Zwischenzeitlich existieren auf dem Markt allerdings Reinigungs- und Desinfektions-Geräte, in denen die Instrumente aufbereitet werden, bevor sie einer Ölpflege unterzogen werden. Die maschinelle Aufbereitung bringt deutliche Vorteile gegenüber einem manuellen Pflegen der Instrumente mit sich, da nur ein maschinelles Verfahren eine sichere und reproduzierbare Reinigung und Pflege ermöglicht.

Die bislang bekannten Geräte können allerdings in der Regel lediglich für einzelne Aufbereitungsschritte benutzt werden, sodass jeweils separat eine Reinigung, eine Pflege und eine Sterilisation durchgeführt werden muss. Die Gesamtheit der hierfür erforderlichen Geräte nimmt einen relativ großen Platz in Anspruch, wobei für jedes der Geräte jeweils elektrische, pneumatische und fluidische Anschlüsse erforderlich sind. Die Realisierung einer vollständigen maschinellen Aufbereitung zahnärztlicher Instrumente mittels einzelner Geräte ist dementsprechend sehr umständlich und mit einem hohen Kostenaufwand verbunden.

Ein weiterer Nachteil besteht darin, dass die einzelnen Geräte in der Regel nicht untereinander vernetzt sind, weshalb ein Datenaustausch zwischen den Geräten nicht erfolgen kann. Dies führt wiederum zu einem Mehraufwand des Bedienpersonals, da keine durchgängig automatische Dokumentation der Instrumentenaufbereitung erstellbar ist. Ferner müssen in Zwischenschritten die Instrumente von Gerät zu Gerät manuell weiterbefördert werden, was mit einem intensiven Personaleinsatz und einem großen Zeitbedarf verbunden ist.

Aus der Schrift DE 41 27 776 A1 ist ein Reaktorbehälter mit einem Klammerverschluss bekannt, der drei Segmente aufweist, die über Drehgelenke miteinander verbunden sind.

Aus der Schrift DE 94 04 372 U1 ist ein Sterilisationsbehälter bekannt, der mithilfe eines Spannrings mit einem Deckel verschlossen werden kann. Ein ähnlicher Sterilisationsbehälter ist auch aus der Schrift US 5,823,340 bekannt.

Ferner sind aus einem Vorlesungsmaterial der TU München über Apparatebau (Prof. Dr.-Ing. Klaus Strohmeier: "Komponenten des Anlagebaus", 1. Dezember 2002, München und Prof. Dr.-Ing. Klaus Strohmeier: "Beanspruchungsgerechte Anlagensimulation", 1. Dezember 2002, München) Behälterverschltisse bekannt, wobei ein Klammerverschluss Klammern aufweist, die zur Verrieglung eines Deckels um eine senkrechte Drehachse schwenkbar gelagert sind.

Der vorliegenden Erfindung liegt deshalb die Aufgabe zugrunde, ein neuartiges Gerät zum Desinfizieren, Sterilisieren und/oder Pflegen bzw. allgemein zum Aufbereiten von ärztlichen, insbesondere zahnärztlichen Instrumenten anzugeben, mit dem die oben genannten Nachteile vermieden werden.

Die Aufgabe wird durch ein Gerät, welches die Merkmale des unabhängigen Anspruchs aufweist, gelöst.

Die Erfindung wird durch Anspruch 1 definiert. Vorteilhafte Weiterbildungen der Erfindung sind Gegenstand der abhängigen Ansprüche.

Gemäß der vorliegenden Erfindung wird dementsprechend ein Gerät zur Aufbereitung insbesondere von zahnärztlichen Instrumenten angegeben, welches eine intensive Reinigung der Instrumente (außen und innen) mit Reinigungsmitteln und/oder Klarspülern bei unterschiedlichen Temperaturen ermöglicht. Es handelt sich erfindungsgemäß um ein vollautomatisches Gerät, welches lediglich in größeren Zeitabständen Wartungsarbeiten des Personals erfordert. Diese beschränken sich in erster Linie auf das Nachfüllen der Verbrauchsmaterialien, also der Reinigung- und Pflegemittel. Die Aufbereitung der Instrumente selbst hingegen wird ohne Eingriff des Personals durchgeführt, was auf einen vollautomatischen Ablauf inklusive der Möglichkeit der Erstellung einer Dokumentation zurückzuführen ist. Um dieses Ziel zu erreichen, wird ein Pflegegerät vorgeschlagen, welches wie nachfolgend detaillierter beschrieben in zahlreichen Aspekten optimiert wurde.

Ein erster Aspekt der vorliegenden Erfindung betrifft hierbei die Ausgestaltung des Geräts, genauer genommen der Spülkammer, in der die aufzubereitenden zahnärztlichen Instrumente angeordnet werden. Da während der Reinigung und Pflege der Instrumente in der Spülkammer verschiedene Drücke und Temperaturen vorliegen, ist es erforderlich, die Spülkammer als Druck-/Vakuumbehälter auszugestalten, der einfach und sicher verschlossen werden kann.

Um diese Aufgabenstellung zu lösen, wird gemäß einem ersten Aspekt der vorliegenden Erfindung ein Gerät zum Desinfizieren, Sterilisieren und/oder Pflegen von ärztlichen, insbesondere zahnärztlichen Instrumenten, vorgeschlagen, welches eine Spülkammer, einen Deckel zum Verschließen der Spülkammer sowie einen Verriegelungsmechanismus zum Verriegeln des Deckels mit der Spülkammer während des Betriebs aufweist. Hierbei ist erfindungsgemäß vorgesehen, dass der Verriegelungsmechanismus ein oder mehrere Klammern aufweist, welche zur Verriegelung des Deckels mit dem Behälter um eine senkrecht zur Ebene des Deckels ausgerichtete Achse schwenkbar gelagert sind. Die Klammern weisen dabei Klemmvorsprünge auf, die durch einen Bund des Behälters ragen.

Wie später ausführlich erläutert, wird hierdurch ein sehr einfach zu bedienender Verriegelungsmechanismus für den Deckel geschaffen, der gleichzeitig eine sehr sichere Verriegelung der Spülkammer sicherstellt. Durch bestimmte Maßnahmen kann eine Fehlbedienung des Geräts ausgeschlossen und dementsprechend die Betriebssicherheit erhöht werden.

Dabei ist vorzugsweise vorgesehen, dass der Verriegelungsmechanismus zwei Klammern aufweist, welche eine gemeinsame Schwenkachse aufweisen. Hierbei können die beiden Klammern dann an einem der Schwenkachse gegenüberliegenden Ende jeweils an einem Antrieb zum Verschwenken der Klammern gelagert sein. Bei diesem Antrieb handelt es sich um einen Hubzylinder, insbesondere um einen Elektrozylinder, der vorzugsweise in ein Griffelement zum Öffnen und Schließen des Deckels integriert ist. Die Klammern können jeweils eine Schräge aufweisen, welche mit einer an dem Deckel vorgesehenen Schräge zusammenwirkt, sodass beim Schließen der Verriegelungsvorrichtung der Deckel gegen die Kammer gepresst wird. Um eine sichere Verriegelung zu erreichen, können die Klammern hierzu Klemmvorsprünge aufweisen, welche Ausnehmungen durchgreifen, die in einem dem Deckel zugewandten Stirnbereich des Behälters vorgesehen sind.

Letztendlich wird also ein Gerät vorgeschlagen, welches in vollautomatischer, zuverlässiger und reproduzierbarer Weise ein Aufbereiten insbesondere zahnärztlicher Instrumente ermöglicht.

Nachfolgend soll die Erfindung anhand der beiliegenden Zeichnung näher erläutert werden. Es zeigen:
- Figur 1: in Schnittdarstellung eine Prozess- bzw. Spülkammer eines erfindungsgemäßen Geräts zum Desinfizieren, Sterilisieren und/oder Pflegen von zahnärztlichen Instrumenten;
- Figuren 2 und 3: eine mögliche Ausgestaltung einer Spülkammer des Geräts von Figur 1;
- Figuren 4 bis 7: Darstellungen eines an der Oberseite der Spülkammer angeordneten Deckels, der mit der Spülkammer verriegelt werden kann;
- Figur 8: die Ausgestaltung des Systems zur Entnahme von Medien aus Vorratsbehältern durch die Erzeugung von Unterdruck in der Spülkammer und
- Figur 9: die Vorgehensweise zur automatischen Erkennung der Belegung von Halterungen des Geräts durch aufzubereitende Instrumente.

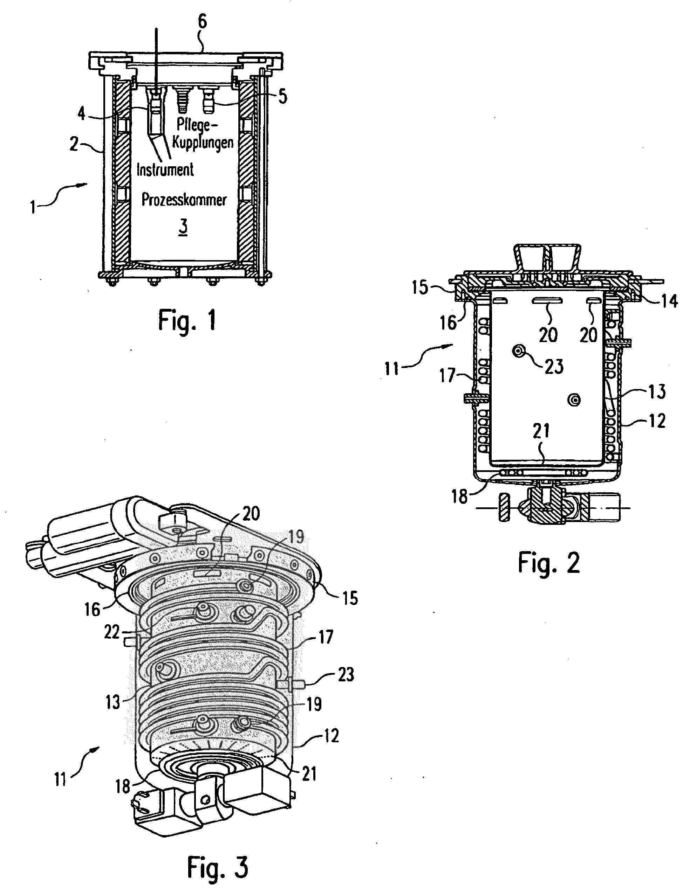

Figur 1 zeigt zunächst schematisch die Ausgestaltung eines erfindungsgemäßen Geräts zum Desinfizieren, Sterilisieren und/oder Pflegen von ärztlichen, insbesondere zahnärztlichen Instrumenten, wobei das Gerät nachfolgend allgemein mit dem Bezugszeichen 1 versehen ist. Zentrales Element des erfindungsgemäßen Pflegegeräts 1 ist ein Druckbehälter 2, der eine Prozess- bzw. Spülkammer 3 umschließt. In dieser Spülkammer 3 sind während des Prozessablaufs die zu reinigenden bzw. pflegenden Instrumente 4 angeordnet. Die Anordnung der Instrumente 4 erfolg hierbei mit Hilfe eines Instrumententrägers, auf dem mehrere Steckplätze bzw. Kupplungen 5 angeordnet sind. Vorzugsweise sind unterschiedliche Kupplungen 5 vorgesehen, sodass Instrumente 4 mit Kupplungssystemen verschiedener Hersteller aufbereitet werden können. Als Instrumententräger dient im vorliegenden Fall der Deckel 6 der Prozesskammer 3, der nachfolgend noch näher beschrieben wird. Dieser Deckel 6 stellt die fluidische Ankopplung der zu reinigenden Instrumente 4 an ein Versorgungssystem sicher. Er wird durch eine nachfolgend detailliert beschriebene Verriegelungsvorrichtung auf den Bund des Druckbehälters 2 geklemmt und gegenüber diesem abgedichtet. Über in den Deckel 6 integrierte Verbindungsrohre können dann die einzelnen Instrumente 4 und deren Kanäle einzeln oder gemeinsam mit einem Reinigungs- und/oder Pflegemittel beaufschlagt werden.

Bevor nunmehr einzelne Komponenten bzw. Elemente des erfindungsgemäßen Geräts detaillierter besprochen werden, soll zunächst allgemein der Prozessablauf bei der Reinigung und/oder Pflege der Instrumente 4 beschrieben werden. Hierbei wird vor dem Start der Aufbereitung die Druckdichtigkeit der Prozesskammer 3 überprüft. Dabei wird sichergestellt, dass der Deckel 6 richtig eingesetzt und mit dem Druckbehälter 2 verriegelt ist. Auch eine korrekte Verbindung der Fluidleitungen zwischen dem Deckel 6 und in dem Bund des Druckbehälters 2 verlaufenden Leitungen wird überprüft.

Zur Wasserversorgung des Geräts 1 wird Leitungswasser vorzugsweise mittels einer Osmose-Anlage mit oder ohne nachgeschalteten Mischbett-Ionenaustauscher filtriert, wobei die gelösten Salze entfernt werden. Das Wasser bei einer Qualität von <15 µS/cm wird in einen geräteseitigen Vorratsbehälter geleitet, wobei der Füllstand über einen Niveauschalter, der als Schwimmerschalter ausgestaltet ist, und die Güte über einen Leitwertsensor kontrolliert wird. Der Einlass in den Vorratsbehälter ist aus hygienischen Gründen mit einer sogenannten freien Fallstrecke ausgestaltet.

Beim Aufbereiten der Instrumente mit Hilfe des erfindungsgemäßen Geräts werden dann nacheinander die folgenden Schritte ausgeführt:

### a) Reinigen

Zunächst wird Wasser aus dem zuvor beschriebenen Vorratsbehälter in die Prozesskammer 3 geleitet, wobei dies über eine Pumpe oder - wie später beschrieben - durch Ansaugen über Vakuum erfolgen kann. In der Prozesskammer 3 wird das Wasser mit Hilfe von Heizelementen auf etwa 45°C aufgeheizt. Dabei wird darauf geachtet, dass die Temperatur nicht oberhalb von 45°C liegt, um ein Koagulieren von Eiweiß zu verhindern. Das Wasser wird ferner mit Hilfe einer Pumpe umgewälzt und über Sprühdüsen, welche an der Mantelfläche des Druckbehälters 2 oder in einem Zentraldom angebracht sind, auf die Außenflächen der Instrumente 4 gerichtet, um diese zu reinigen. Dabei kann das Reinigungswasser durch die Instrumente 4 und/oder die Spraykanäle der Instrumente 4 und/oder zur Außenreinigung durch die Sprühdüsen der Prozesskammer 3 geleitet werden.

Das Aufheizen des Waschmediums kann während der Umwälzung erfolgen, sodass die zu reinigenden Flächen zunächst mit kaltem Waschmedium gereinigt werden. Das Reinigungsmittel kann hierbei in Form von Pulver oder in Tablettenform in die Prozesskammer 3 zugegeben werden oder aus einem entsprechenden Vorratsbehälter zudosiert werden. Die verschiedenen Möglichkeiten zum Dosieren des Reinigungsmittels werden nachfolgend beschrieben. Das Waschmedium kann dabei aus Tensiden bzw. Phosphaten bestehen und einen ph-Wert oberhalb von 10 aufweisen. Zur Beendigung des Waschvorgangs wird das Wasser aus dem Druckbehälter 2 abgelassen.

### b) Klarspülen - Neutralisation

In einem darauffolgenden Schritt wird dann das Wasser aus dem Vorratsbehälter in die Prozesskammer 3 geleitet und nun auf ca. 45°C bis 60°C aufgeheizt. Während des Umwälzenz des Wassers wird aus einem weiteren Vorratsbehälter Klarspüler bzw. Neutralisator zudosiert. Alternativ kann aufgrund der höheren Temperatur im Vergleich zu dem Schritt a) nunmehr auch eine zweite Komponente einer Reinigungstablette aufgelöst werden. Die Flüssigkeit wird wiederum parallel oder zeitversetzt bzw. im Intervallbetrieb durch die Instrumente 4 und die Spraykanäle geleitet bzw. über die Sprühdüsen auf die Außenflächen der Instrumente 4 gerichtet. Als Klarspüler bzw. Neutralisator kommt insbesondere Phosphorsäureester mit einem ph-Wert von 3 bis 5 zur Anwendung.

Die Flüssigkeit kann wiederum aus dem Druckbehälter in die Kanalisation abgelassen werden oder verbleibt im Behälter, um beim späteren Pflegevorgang aus den Instrumenten 4 austretendes überschüssiges Pflegemittel aufzunehmen bzw. um die ölige Instrumentenaußenfläche mit warmer Flüssigkeit kurz abzuspülen. In diesem Fall wird die Flüssigkeit erst nach dem Pflegevorgang abgelassen, wobei es hilfreich sein kann, die Instrumente 4 mit Druckluft zu beaufschlagen, um ein Eindringen von Sprühwasser in das Innere der Instrumente 4 zu verhindern.

### c) Pflegen

In einem dritten Schritt wird aus einem Pflegemittelvorratsbehälter Pflegemittel in das Instrumenteninnere geleitet, sodass die Getriebe und Lagerstellen geschmiert werden. Das Pflegemittel kann dabei in flüssiger Form als Öl oder aus einer Druckdose in einen Druckluftstrahl injektiert werden. Es ist auch möglich, das Öl über das in der Druckdose enthaltene Treibmittel aufzuschäumen und das Instrumenteninnere mit diesem Öl-Luft-Schaum zu füllen. Die Luftbläschen kollabieren in diesem Fall verhältnismäßig rasch, sodass das Öl im gesamten Instrumenteninneren einen gleichförmigen dünnen Ölfilm bildet. Als Schmierstoffe kommen biologisch abbaubare Fettsäure-Esteröl/Weißöl-Gemische zur Anwendung.

### d) Abspülen

Nachdem zuvor beschriebenen Pflegevorgang können die Instrumente an der Außenfläche mit der noch im Behälter befindlichen Klarspülflüssigkeit abgespült werden. Alternativ hierzu wird über eine Pumpe frisches Wasser aus dem Vorratsbehälter der Prozesskammer 3 zugeführt und über die Sprühdüsen auf die Instrumentenaußenflächen gerichtet.

### e) Sterilisation - Vorvakuum

Zum Sterilisieren der Instrumente wird der Prozesskammer 3 frisches Wasser aus dem Vorratsbehälter zugeführt. In der Prozesskammer 3 ist zur Entlüftung eine Vakuumeinrichtung angeschlossen, wobei der Druck innerhalb der Prozesskammer 3 überwacht bzw. registriert wird.

Mit Hilfe der Vakuumeinrichtung wird die Luft aus der Prozesskammer 3 abgesaugt. Das Vakuum wird durch Aufheizen des Wassers über Heizelemente bis auf Atmosphärendruck abgebaut. Die Prozesskammer 3 wird dann mit Wasserdampf befüllt, wobei sich dieser Vorgang je nach Sterilisationsprogramm mehrmals wiederholen kann.

Das verdampfte Wasservolumen kann bei jedem Vakuumzyklus nachgefüllt werden, wobei alternativ hierzu auch die gesamte für die Dampferzeugung erforderliche Wassermenge gleich zu Beginn des Sterilisationszyklus in die Prozesskammer 3 eingebracht werden kann.

Alternativ zur Dampferzeugung über in der Prozesskammer 3 befindliche Heizelemente kann Wasserdampf zum Druckausgleich bei der Entlüftung bzw. zur Sterilisation auch aus einem außerhalb der Prozesskammer 3 befindlichen Dampfdruckkessel zugeführt werden.

### f) Trocknung und Kühlung

Nach Abschluss der Sterilisation werden die Instrumente 4 getrocknet, in dem der in der Prozesskammer 3 befindliche Wasserdampf zur Kondensation gebracht wird. Dies wird dadurch erzielt, dass die Behälterwand oder in dem Behälter befindliche Elemente gekühlt werden, beispielsweise indem aus dem Vorratshälter entnommenes Wasser durch sie geleitet wird. Dabei kann das Wasser kontinuierlich oder intervallförmig zugeführt werden. Nach Beendigung des Kühlvorgangs wird das Wasser abgeleitet. Da nunmehr innerhalb der Kamer 3 eine Temperatur unterhalb von 50°C vorliegt, kann der Deckel 6 geöffnet werden. Der Aufbereitungszyklus für die Instrumente 4 ist hierdurch abgeschlossen.

Aus der obigen Schilderung ergibt sich, dass mit dem erfindungsgemäßen Gerät 1 eine vollautomatische Aufbereitung zahnärztlicher Instrumente ermöglicht wird. Eingriffe durch Bedienpersonal sind nicht erforderlich, sodass ein sehr komfortables System vorliegt. Einzelne Details dieses Geräts sollen nachfolgend näher beschrieben werden.

Dabei soll zunächst anhand der Figuren 2 und 3 eine vorteilhafte Ausführungsform einer Prozesskammer bzw. eines Spülbehälters erläutert werden. Der Behälter wurde hierbei optimiert, um die Zykluszeiten für die Aufwärm- und Abkühlphasen während der Aufbereitungsschritte der Instrumente zu reduzieren.

Die allgemein mit dem Bezugszeichen 11 bezeichnete Prozess- bzw. Spülkammer ist hierzu aus einem tiefgezogenen Außenbehälter 12, der als Druckbehälter ausgerührt ist, und einem tiefgezogenen dünnwandigen Innenbehälter 13 aufgebaut. Alternativ können beide Behälter 12 und 13 auch als Schweißkonstruktion aus mehreren Teilen ausgeführt werden. Der Außenbehälter 12 ist hierbei über Schraubverbindungen 14 mit dem Behälterbund 15 verschraubt. Zwischen Außenbehälter 12 und Behälterbund 15 ist eine Dichtung 16 in Form eines umlaufenden Dichtungsrings ausgebildet.

Der Innenbehälter 13 ist am Behälterbund 15 eingehängt und kann zum Reinigen der Spülkammer oder zum Austausch der Heizung einfach nach oben entnommen werden. Dabei wird die Lage des Innenbehälters 13 über am Behälterbund 15 ausgeführte Stifte und entsprechende Aussparungen am Innenbehälter 13 festgelegt. Hierdurch ist sichergestellt, dass der Innenbehälter 13 im Hinblick auf die Öffnungen für die Reinigungsdüsen zum Reinigen der - nicht dargestellten - Instrumente richtig positioniert ist.

In dem Zwischenraum zwischen Außenbehälter 12 und Innenbehälter 13 befinden sich eine Kühlwendel 17 sowie eine Heizwendel 18. Da - wie nachfolgend noch näher erläutert - der Innenbehälters 13 gegenüber dem Außenbehälter 12 nicht abgedichtet ist, befinden sich die Kühlwendel 17 und die Heizwendel 18 mit ihrer gesamten Fläche innerhalb der Spülkammer, wodurch eine optimale Wärmeübertragung bzw. Temperaturkopplung gewährleistet ist. Die Oberfläche der Kühlwendel 17 beträgt bei dem dargestellten Ausführungsbeispiel etwas das 2,5-fache der zylindrischen Außenmantelfläche. Es steht also eine sehr große Fläche zur Wärmeübertragung zur Verfügung. Die Kühlwendel 17 wird mittels angelöteten bzw. angeschweißten Adaptern 19 mit dem Außenbehälter 12 verschraubt. Ein Verschweißen oder Verlöten am Außenbehälter 12, wie dies bei einer außen liegenden Kühlwendel erforderlich wäre, ist bei dieser Lösung nicht erforderlich. Die Heizwendel 18, welche im dargestellten Ausführungsbeispiel an der Unterseite des Innenbehälters 13 angeordnet ist, könnte alternativ auch als Flächenheizelement am Boden innen oder außen angebracht werden.

Um eine effektive Dampfzirkulation zwischen dem Außenbehälter 12, genauer gesagt zwischen dem Zwischenraum zwischen dem Innen- und Außenbehälter und dem Innenbereich des Innenbehälters 13 zu erreichen, sind im oberen Bereich des Innenbehälters 13 Durchbrüche bzw. Durchgangsöffnungen 20 vorgesehen. Weitere derartige Durchbrüche befinden sich im Bereich der Reinigungsdüsen sowie am Boden. Diese Bodenperforation 21 besitzt gleichzeitig eine Siebfunktion, um das Eindringen von Kleinteilen in den Ablauf des Behälters zu verhindern.

Ferner sind an der Außenfläche des Innenbehälters 13 Temperaturfühler 22 angeordnet. Diese sind abgewinkelt ausgeführt und werden aufgrund ihrer Anordnung von mechanischen Beschädigungen geschützt.

Die Reinigungsdüsen 23 werden über eine Dichtung mit dem Außenbehälter 22 verschraubt und sprühen über eine entsprechende Öffnung am Innenbehälter 13 auf die Instrumente. Die Befestigungsbereiche der verschiedenen Anschraubpunkte sind jeweils geprägte, plane Flächen und können einfach zum jeweiligen Bauteil beispielsweise über O-Ringe, Flachdichtungen, usw. abgedichtet werden. Die Heizwendel 18 am Behälterboden besitzt einen kleineren Außendurchmesser als der Innendurchmesser der Kühlwendel 17 und kann dementsprechend bei Bedarf ohne Demontage der Kühlwendel 17 entfernt bzw. ausgebaut werden.

Selbstverständlich könnte die Prozesskammer auch anders gestaltet, beispielsweise doppelwandig ausgeführt sein, wobei dann der Hohlraum zwischen den Wänden zum Durchleiten eines Kühlmediums genutzt wird.

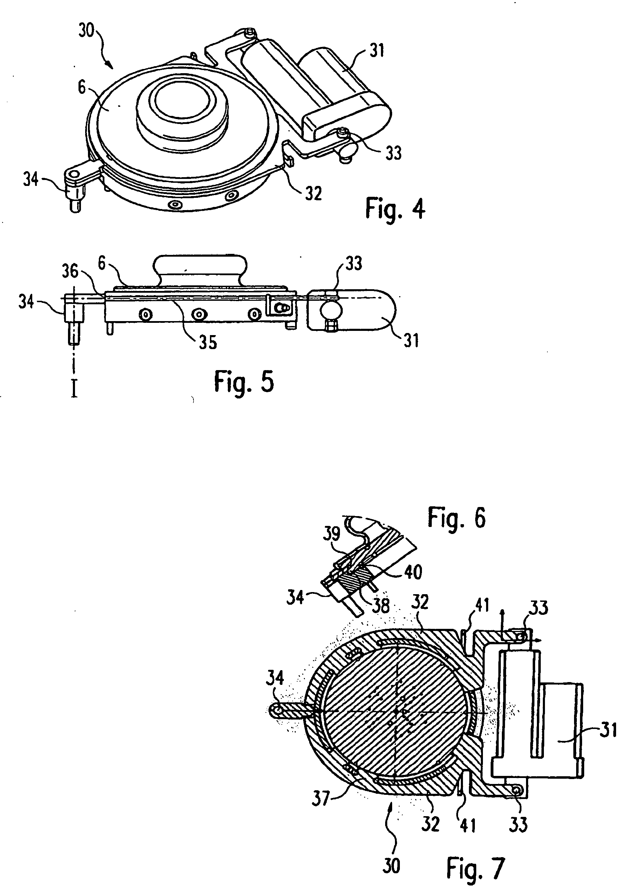

Ein ordnungsgemäßer Ablauf der zuvor beschriebenen Schritte beim Aufbereiten der Instrumente ist offensichtlich nur dann möglich, wenn die Prozesskammer 3 druck-/vakuumdicht verschlossen wird. Hierfür ist ein spezieller Deckel mit einem Verriegelungsmechanismus vorgesehen, der in den Figuren 4 bis 7 dargestellt ist und nachfolgend näher erläutert werden soll.

Der Verriegelungsmechanismus 30 weist hierbei zunächst einen Hubzylinder 31 auf, der vorzugsweise als Elektrozylinder ausgebildet ist und interne oder externe Endschalter aufweist. An dem Hubzylinder 31 sind zwei bogenförmige Verriegelungsklammern 32 über Gelenke 33 befestigt, wobei die Klammern 32 an ihrem gegenüberliegenden Ende an einem Lagerbolzen 34 angeordnet sind. Dieser Lagerbolzen 34 definiert eine senkrecht zur Ebene des Deckels 6 ausgerichtete Schwenk- bzw. Drehachse I, um welche beide Klammern 32 geschwenkt werden können.

Beide Klammern 32 sind dabei in einer Nut 35 im Behälterbund 36 gelagert. Beim Schließen der Klammern 32 ragen Klemmvorsprünge bzw. Klauen 37 der Klammern 32 durch den Behälterbund 36 und drücken über eine Schräge 38 den Behälterdeckel 6 über eine umlaufende oder partielle Nut 39 auf eine stirnseitige Dichtung 40, die beispielsweise als umlaufender O-Ring ausgeführt sein kann. Die Nut 39 des Behälterdeckels 6 weist dabei die gleiche Schräge wie die Klauen 37 der Verriegelungsklammern 32 auf, sodass ein Zusammenwirken beider Elemente sichergestellt ist.

Wird als Hubzylinder 31 ein Elektrozylinder verwendet, so ist der Deckel 6 sicher verschlossen, da sich aufgrund der Selbsthemmung der vorzugsweise verwendeten Lineargewindewelle die Klammern 32 selbst bei Stromausfall nicht öffnen können. Sollte zu Beginn des Schließvorgangs der Deckel nicht auf der Behälterdichtung 40 anliegen und eine größere Linearbewegung zum Schließen erforderlich sein, so können die Klauen 37 der Klammern 32 und die Deckelnut 39 auch eine steilere Schräge aufweisen.

Vorzugsweise sind die beiden Klammern 32 derart ausgestaltet, dass beim Öffnen des Behälters eine der beiden Klammern 32, welche einen geringeren Reibwert aufweist, bis zu einem der beiden Anschläge 41 ausschwenkt. Erst dann öffnet auch die zweite Klammer 32. Die Endstellungen der Klammern 32 im geöffneten und geschlossenen Zustand können dabei durch externe Endschaltung oder durch interne Endschalter im Elektrozylinder 31 bzw. entsprechende Kombinationen hiervon detektiert werden.

Um Beschädigungen durch einen nicht ordnungsgemäß eingesetzten Behälterdeckel 6 zu verhindern, kann bei Verwendung eines Elektrozylinders 31 dieser mit einer Strombegrenzung ausgestattet sein. Um ferner sicherzustellen, dass der Deckel 6 ordnungsgemäß eingesetzt wurde, kann im Behälter kurzeitig ein Unterdruck erzeugt und durch einen Drucksensor erfasst werden. Erst wenn der Unterdruck einen gewissen Schwellenwert erreicht hat, was nur der Fall ist, wenn der Deckel 6 ordnungsgemäß eingesetzt wurde, kann der Schließvorgang stattfinden.

Es handelt sich hierbei um ein einfaches, selbstzentrierendes System, bei dem durch die Befestigung der Verriegelungsklammern 32 am Hubzylinder 31 gleichzeitig eine Montage des Gesamtsystems erfolgt. Ferner wird ein günstiger, direkter Kraftfluss vom Deckel 6 über die Klammern 32 auf den Behälterbund erzielt. Beim Verriegeln des Deckels 6 wird gleichzeitig dieser gegen die Dichtung gepresst, sodass eine hohe Dichtigkeit sowohl bei Unter- als auch bei Überdruck erzielt wird. Ferner wird durch die stirnseitige Abdichtung des Behälters beim Öffnen des Deckels eventuell vorhandener Druck sofort abgebaut. Dadurch kann der Deckel 6 nicht nach oben beschleunigt werden und entsprechend nicht zu einer Gefährdung des Bedieners führten.

Dem oben geschilderten Verfahrensablauf ist entnehmbar, dass bei der Aufbereitung der Instrumente verschiedene chemische Substanzen zur Reinigung, Pflege und Desinfektion zum Einsatz kommen. Die Substanzen werden in unterschiedlichen Zustandsformen (fest, pulvrig oder flüssig) und Verpackungen (Spraydosen, Flüssigdispenser-Flaschen oder Tabletten) angeboten und eingesetzt. Eine Kennzeichnung der unterschiedlichen Produkte erfolgt dabei üblicherweise durch entsprechende Aufdrucke auf der Verpackung bzw. dem Gefäß.

Neben Vorratsbehältern in Form von Spraydosen kommen bei dem erfindungsgemäßen Gerät auch Behälter zum Einsatz, welche zuzuführende Medien in flüssiger Form bereitstellen. Insbesondere wird bei dem eingangs beschriebenen Prozessablauf Wasser VE-Wasser, Flüssigreiniger und Klarspüler zugeführt. Um diese Medien in die Prozesskammern zu fördern war in der Vergangenheit pro Medium eine separate Förderpumpe eingesetzt worden. Diese Art der Medienförderung hat allerdings einen großen Aufwand an Material und Kosten zur Folge.

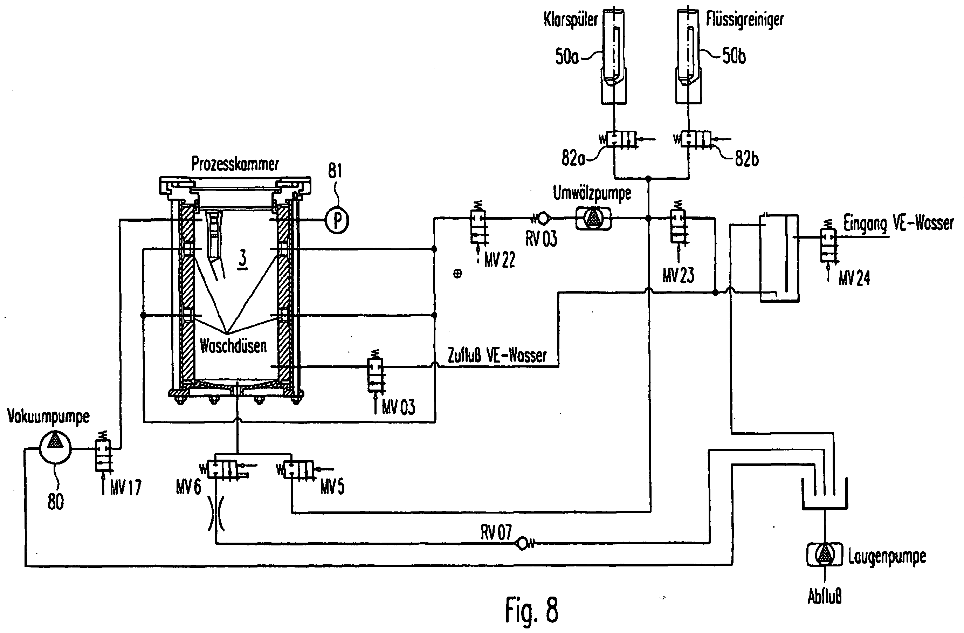

Figur 8 zeigt nunmehr eine Lösung dieses Problems welche darauf beruht, innerhalb der Prozesskammer 3 ein Vakuum zu erzeugen. Hierfür kann die Vakuumpumpe 80 eingesetzt werden, welche ohnehin zur Durchführung der verschiedenen Reinigungs- und Pflegeschritte erforderlich ist. Diese Pumpe 80 erzeugt nunmehr einen Unterdruck von vorzugsweise etwa 500 mbar innerhalb der Prozesskammer, wobei dieser Druck möglichst genau sein sollte, um eine exakte Dosierung der zuzuführenden Medien sicherzustellen. Zur Kontrolle des Vakuums innerhalb der Prozesskammer 3 wird der ebenfalls üblicherweise bereits vorhandene Drucksensor 81 eingesetzt.

Soll nunmehr ein bestimmtes Medium zugeführt werden, so wird für eine festgelegte Zeitspanne, die zuvor experimentell ermittelt wurde, das zu dem zugehörigen Vorratsbehälter 50a, 50b gehörende Ventil 82a bzw. 82b geöffnet, sodass aufgrund des Unterdrucks das zugehörige Medium in die Prozesskammer 3 gesaugt wird. Durch Freischalten des zugehörigen Ventils kann dementsprechend das gewünschte Medium in die Prozesskammer 3 gefördert werden.

Die Dosierung des geförderten Mediums kann nunmehr beispielsweise dadurch erfolgen, dass das Zulaufventil für eine vorab definierte Zeit geöffnet wird. Bei dieser zeitgesteuerten Variante muss dementsprechend zuvor ermittelt werden, welche Medienmenge innerhalb welchen Zeitraums bei Anliegen des definierten Unterdrucks in die Kammer 3 gefördert wird.

Alternativ wäre auch eine druckgesteuerte Variante möglich. Hierbei wird nach Einleiten des zu dosierenden Mediums in die Prozesskammer 3 das zugehörige Ventil so lange geöffnet, bis ein zweiter, geringerer Unterdruck in der Prozesskammer erzielt wird.

Bei höheren Unterdrücken erfolgt der Zulauf des zu dosierenden Mediums rascher, während er sich hingegen bei geringeren Unterdrucken verlangsamt. Es hat sich herausgestellt, dass ein Unterdruck von etwa 500 mbar zu einem sehr guten Kompromiss führt, da hierbei sowohl eine rasche Zuführung bzw. Abfüllung als auch eine hohe Genauigkeit bei der Dosierung ermöglicht ist.

Durch die erfindungsgemäße Lösung, bei der die Förderung der Medien durch das an der Prozesskammer 3 anliegende Vakuum erfolgt, kann auf den Einsatz separater Förderpumpen verzichtet werden. Lediglich diejenigen Medien werden der Prozesskammer zugeleitet, deren Zulaufventil zur Prozesskammer bei anliegendem Vakuum geöffnet ist. Die Lösung ist dementsprechend deutlich kostengünstiger als bislang bekannte Ausgestaltungen, wobei trotz allem eine rasche aber genaue Dosierung der Medien erzielt wird.

Auch der nachfolgend beschriebene Erfindungsgedanke befasst sich mit der Zuführung bzw. Dosierung der während des Prozessablaufs genutzten Medien. Dabei soll nunmehr sichergestellt werden, dass die Menge des jeweils zugeführten Mediums an die Anzahl der aufzubereitenden Instrumente angepasst ist. Je nach Anzahl der belegten Halterungen bzw. Pflegekupplungen soll dementsprechend die Medienmenge angepasst werden, weshalb erforderlich ist, zu erkennen bzw. einzustellen, wie viele Instrumente sich tatsächlich innerhalb der Prozesskammer befinden.

Aus dem Stand der Technik ist bislang bekannt, die Erkennung der Belegung über verschiedene Arten von Schaltern durchzuführen, über welche dann die zugehörigen Ventile angesteuert wurden. Bei einer geschlossenen Kammer hingegen, wie sie vorzugsweise bei dem vorliegenden Gerät vorliegt, ist eine entsprechende Erkennung der Instrumente schwieriger. Aus der EP 1 749 502 A ist ferner eine Lösung bekannt, bei der die zugehörige Instrumentenzuleitung mit Druck beaufschlagt wird, wobei diese Lösung allerdings mehrere Drucksensoren erfordert.

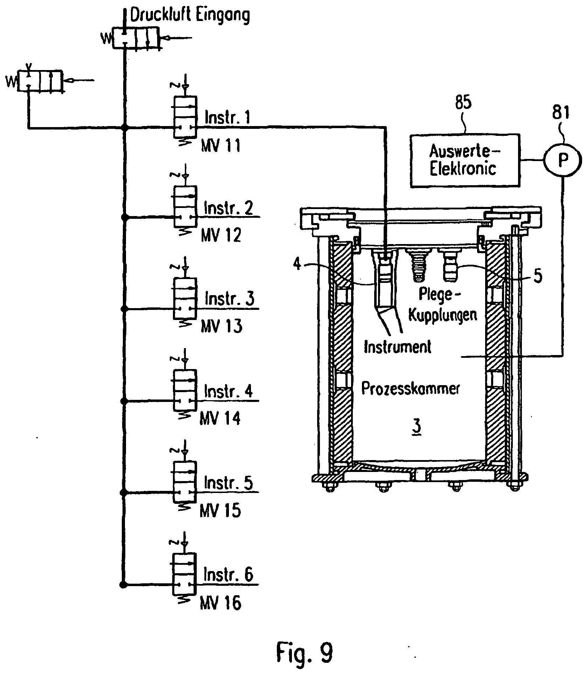

Eine im Vergleich dazu einfachere Lösung ist in Figur 9 dargestellt, welche auf dem Gedanken beruht, den für den Sterilisationsvorgang ohnehin benötigten Drucksensor 81, der innerhalb der Prozesskammer 3 angeordnet ist, für die Belegungserkennung zu verwenden.

Bei der erfindungsgemäßen Lösung wird in der Prozesskammer 3 ein Überdruck erzeugt und anschließend das zu überprüfende Kupplungsventil MV11 - MV16 geöffnet. Das Ventil MV11 - MV16 entlüftet dann atmosphärisch, wobei anhand des sich in der Prozesskammer 3 einstellenden Druckabfalls innerhalb einer vorherbestimmten Zeitspanne festgestellt werden kann, ob die angesprochene Kupplung 5 durch ein Instrument 4 belegt ist oder nicht. Liegt ein geringer Druckabfall vor, so bedeutet dies, dass die Kupplung 5 belegt ist. Ein großer Druckabfall hingegen lässt auf eine nicht belegte Kupplung 5 schließen. Auf diese Weise können dann die Kupplungen 5 nacheinander abgefragt werden.

Die Vorgehensweise ist schematisch in Figur 9 dargestellt, wobei zunächst über die Ventile MV10 und MV11 Druckluft in die Prozesskammer 3 geführt wird, um einen Überdruck von beispielsweise 0,5 bis 2 bar, typischerweise 1 bar zu erzeugen. Anschließend wird über die Ventile MV11 und MV31 eine definierte Zeit von 1 bis 10 Sekunden, beispielsweise 2 Sekunden Luft aus der Prozesskammer 3 abgelassen und der Druckabfall in der Prozesskammer bzw. der Gradient des Druckabfalls ermittelt. In einer Auswerteelektronik 85 sind die Werte von unbelegten Kupplungen hinterlegt, sodass anhand der entstandenen Druckdifferenz über die Auswertelektronik 85 festgestellt werden kann, ob die entsprechende Kupplung 5 mit einem Instrument 4 bestückt ist oder nicht. Dieser Vorgang wird dann so oft wiederholt, bis alle Kupplungen 5 abgefragt wurden.

Es ist unmittelbar erkennbar, dass zur Durchführung der erfindungsgemäßen Erkennung der Kupplungsbelegung ein einzelner Drucksensor ausreichend ist, weshalb das Verfahren sehr einfach und kostengünstig realisiert werden kann. Ein weiterer Vorteil besteht darin, dass einige dentale Instrumente aus Hygienegründen mit einem System, beispielsweise einem Ventil ausgestattet sind, das eine Rücksaugung von Spraywasser in das Instrument und die Schlauchleitung im Einsatz verhindert. Bei einer Druckbeaufschlagung von der Spannzangenseite her, wie dies erfindungsgemäß vorgesehen ist, schließt dieses Rückschlagventil und führt zu einer signifikanten Reduzierung des Druckabfalls in der Prozesskammer 3. Die Belegung der Kupplungen mit derartigen Instrumenten kann dementsprechend sicher erkannt werden. Im Gegensatz dazu erfolgt bei der Lösung der zuvor genannten EP 1 749 502 A die Druckbeaufschlagung von der Kupplungsseite her, was zur Folge hat, dass das Rückschlagventil lediglich als Strömungshindernis dient, sodass hier eine Erkennung der Belegung der Kupplung deutlich schwieriger ist.

Alternativ zu der zuvor beschriebenen Vorgehensweise wäre es auch denkbar, die Anwesenheit der Instrumente mit Hilfe eines kapazitiven oder induktiven Messsystems zu ermitteln. Beispielsweise könnte in der Nähe der Kupplungen eine Spule angeordnet werden. Befindet sich nunmehr ein Instrument, bei dem es sich um einen metallischen Gegenstand handelt, in der Nähe der Spule, so ergibt sich ein Einfluss auf die Induktivität der Spule. Dieser kann gemessen werden, wodurch die Belegung der Kupplung festgestellt werden kann. In vorteilhafter Weise kann die Elektronik zum Auswerten bzw. Ermitteln der Anwesenheit der Instrumente an den Mitteln zum Verteilen der Medien angeordnet werden. Dies hat zur Folge, dass lediglich ein Erkennungssystem erforderlich ist.

In einer Weiterbildung kann ferner vorgesehen sein, dass sich das System selbst abgleicht, indem beispielsweise zunächst die Induktivitätswerte in einem Zustand ermittelt werden, in dem keine Belegung der Kupplungen vorliegt bzw. ein Dummyinstrument angeordnet ist.

Alternativ zu der zuvor beschriebenen induktiven Messung könnte selbstverständlich auch ein kapazitives Element benutzt werden, dessen Kupplung mit dem aufzubereitenden Instrument wiederum einen Einfluss auf die Kapazität mit sich bringt.

Insgesamt wird dementsprechend gemäß der vorliegenden Erfindung ein Konzept für ein Gerät zum Desinfizieren, Sterilisieren und/oder Pflegen von ärztlichen, insbesondere zahnärztlichen Instrumenten vorgeschlagen, weiches in zahlreichen Details optimiert wurde, um eine vollautomatische, zuverlässige und reproduzierbare Aufbereitung dentaler Instrumente zu ermöglichen.

## Patentansprüche

1. Gerät (1) zum Desinfizieren, Sterilisieren und/oder Pflegen von ärztlichen, insbesondere zahnärztlichen Instrumenten (4), aufweisend
• eine Spülkammer (3),
• einen Deckel (6) zum Verschließen der Spülkammer, sowie
• einen Verriegelungsmechanismus (30) zum Verriegeln des Deckels (6) mit der Spülkammer (3) während des Betriebs,
wobei der Verriegelungsmechanismus (30) zwei Klammern (32) aufweist, welche zur Verriegelung des Deckels (6) mit dem Behälter (3) um eine senkrecht zur Ebene des Deckels (6) ausgerichtete Achse (I) schwenkbar gelagert sind,
**dadurch gekennzeichnet,**
**dass** die Klammern (32) Klemmvorsprünge bzw. Krallen (37) aufweisen, welche zum Verriegeln des Deckels (6) in einem Stirnbereich des Behälters (3) befindliche Ausnehmungen durchgreifen.

2. Gerät nach Anspruch 1,
**dadurch gekennzeichnet,**
**dass** der Verriegelungsmechanismus (30) zwei Klammern (32) aufweist, welche eine gemeinsame Schwenkachse (I) aufweisen.

3. Gerät nach Anspruch 2,
**dadurch gekennzeichnet,**
**dass** die beiden Klammern (32) an einem der Schwenkachse (I) gegenüberliegenden Ende jeweils an einem Antrieb zum Verschwenken der Klammern (32) gelagert sind, wobei vorzugsweise der Antrieb in ein Griffelement zum Öffnen und Schließen des Deckels (6) integriert ist.

4. Gerät nach Anspruch 3,
**dadurch gekennzeichnet,**
**dass** es sich bei dem Antrieb um einen Hubzylinder (31), insbesondere um einen Elektrozylinder handelt.

5. Gerät nach einem der vorherigen Ansprüche,
**dadurch gekennzeichnet,**
**dass** die Klammern (32) jeweils eine Schräge (38) aufweisen, welche mit einer an dem Deckel (6) vorgesehenen Schräge zusammenwirken.

6. Verfahren zum Erkennen der Belegung von Instrumenten-Halterungen (5) eines Geräts (1) zum Desinfizieren, Sterilisieren und/oder Pflegen von ärztlichen, insbesondere zahnärztlichen Instrumenten (4), wobei das Gerät eine Spülkammer (3) und in der Spülkammer (3) befindliche Halterungen (5) zur Aufnahme der Instrumente (4) aufweist, welche jeweils ein Kupplungsventil aufweisen bzw. mit einem Ventil (MV11 - MV 16) gekoppelt sind,
**dadurch gekennzeichnet,**
**dass** zum Erkennen der Belegung der Halterungen (5) durch Instrumente (4) ein Überdruck in der Spülkammer (3) erzeugt wird, anschließend das Ventil (MV11 - MV 16) einer Halterung (5) geöffnet und anhand des sich nachfolgend ergebenden Druckabfalls in der Kammer (3) die Belegung der Halterung (5) durch ein Instrument (4) festgestellt wird.

7. Verfahren nach Anspruch 6,
**dadurch gekennzeichnet,**
**dass** aufeinanderfolgend die Belegung jeder einzelnen Halterung (5) durch ein Instrument (4) ermittelt wird.

## Claims

1. A device for disinfecting, sterilizing and/or maintaining medical, in particular dental, instruments (4), having
• a rinsing chamber (3),
• a cover (6) for closing the rinsing chamber, and
• a sealing mechanism (30) for providing a seal between the cover (6) and the rinsing chamber (3) during operation,
wherein the sealing mechanism (30) has two clamps (32), which are mounted in a pivotable fashion about an axis (I) aligned perpendicularly with respect to the plane of the cover (6) in order to provide a seal between the cover (6) and the container (3),
**characterized in that**
the clamps (32) have clamping projections or claws (37) which, for the purpose of sealing the cover (6), grip through recesses situated in an end region of the container (3).

2. A device according to claim 1,
**characterized in that**
the sealing mechanism (30) has two clamps (32), which have a common pivot axis (I).

3. A device according to claim 2,
**characterized in that**
the two clamps (32) are, at an end opposing the pivot axis (I), each mounted on a drive for pivoting the clamps (32), the drive preferably being integrated into a handle for opening and closing the cover (6).

4. A device according to claim 3,
**characterized in that**
the drive is a lift cylinder (31), in particular an electric cylinder.

5. A device according to one of the previous claims,
**characterized in that**
the clamps (32) each have a bevel (38), which bevels interact with a bevel provided on the cover (6).

6. A method for identifying the occupancy of instrument holders (5) in a device (1) for disinfecting, sterilizing and/or maintaining medical, in particular dental, instruments (4), wherein the device has a rinsing chamber (3) and holders (5), which are situated in the rinsing chamber (3), serve for receiving the instruments (4) and each have a coupling valve or are each coupled to a valve (MV11-MV16),
**characterized in that**
a positive pressure is generated in the rinsing chamber (3) for identifying the occupancy of the holders (5) by instruments (4), the valve (MV11-MV16) of a holder (5) is subsequently opened and the occupancy of the holder (5) by an instrument (4) is determined on the basis of the subsequent pressure drop in the chamber (3).

7. A method according to claim 6,
**characterized in that**
the occupancy of every single holder (5) by an instrument (4) is established in succession.

## Revendications

1. Dispositif (1) de désinfection, de stérilisation et/ou d'entretien d'instruments (4) médicaux, en particulier dentaires, présentant
- une chambre de rinçage (3),
- un couvercle (6) pour fermer la chambre de rinçage, et
- un mécanisme de verrouillage (30) pour verrouiller le couvercle (6) avec la chambre de rinçage (3) pendant le fonctionnement,
dans lequel le mécanisme de verrouillage (30) présente deux agrafes (32) qui sont placées de manière à pouvoir pivoter, pour le verrouillage du couvercle (6) avec le récipient (3), autour d'un axe (I) orienté perpendiculairement au plan du couvercle (6),
**caractérisé en ce**
**que** les agrafes (32) présentent des saillies de serrage ou des griffes (37) qui s'engagent dans des évidements se trouvant dans une zone frontale du récipient (3) pour verrouiller le couvercle (6).

2. Dispositif selon la revendication 1,
**caractérisé en ce**
**que** le mécanisme de verrouillage (30) présente deux agrafes (32) qui présentent un axe de pivotement (I) commun.

3. Dispositif selon la revendication 2,
**caractérisé en ce**
**que** les deux agrafes (32) sont placées sur l'une des extrémités opposées à l'axe de pivotement (1), respectivement sur un système d'entraînement pour faire pivoter les agrafes (32), dans lequel de préférence, le système d'entraînement est intégré dans un élément formant poignée pour ouvrir et fermer le couvercle (6).

4. Dispositif selon la revendication 3,
**caractérisé en ce**
**que** le système d'entraînement est un cylindre de levage (31), en particulier un cylindre électrique.

5. Dispositif selon l'une des revendications précédentes,
**caractérisé en ce**
**que** les agrafes (32) présentent respectivement un chanfrein (38) qui coopère avec un chanfrein prévu sur le couvercle (6).

6. Procédé de détection de l'occupation de supports d'instruments (5) d'un dispositif (1) de désinfection, de stérilisation et/ou d'entretien d'instruments (4) médicaux, en particulier d'instruments dentaires, dans lequel le dispositif présente une chambre de rinçage (3) et des supports (5) se trouvant dans la chambre de rinçage (3) pour la réception des instruments (4), qui présentent respectivement une soupape d'embrayage ou sont couplés à une soupape (MV11-MV16),
**caractérisé en ce**
**que** pour la détection de l'occupation des supports (5) par des instruments (4), une surpression est générée dans la chambre de rinçage (3), ensuite la soupape (MV11-MV16) d'un support (5) est ouverte et sur la base de la chute de pression qui en résulte ensuite dans la chambre (3), l'occupation du support (5) par un instrument (4) est déterminée.

7. Procédé selon la revendication 6,
**caractérisé en ce**
**que** par la suite, l'occupation de chaque support (5) individuel par un instrument (4) est déterminée.
